Europäisches Patentamt

(19) European Patent Office

Office européen des brevets

(11) Publication number: **0 011 934**
**B1**

(12) **EUROPEAN PATENT SPECIFICATION**

(45) Date of publication of patent specification: **26.09.84**

(21) Application number: **79302450.6**

(22) Date of filing: **05.11.79**

(51) Int. Cl.³: **A 61 N 1/36**

(54) **Body stimulator.**

(30) Priority: **06.11.78 US 957826**
**06.11.78 US 957827**
**06.11.78 US 957828**

(43) Date of publication of application:
**11.06.80 Bulletin 80/12**

(45) Publication of the grant of the patent:
**26.09.84 Bulletin 84/39**

(84) Designated Contracting States:
**GB IT NL SE**

(56) References cited:
**EP-A-0 000 983**
**EP-A-0 000 989**
**DE-A-2 619 001**
**FR-A-2 374 024**
**GB-A-2 014 858**
**US-A-3 726 285**
**US-A-3 833 005**
**US-A-3 835 865**
**US-A-4 049 004**

(73) Proprietor: **MEDTRONIC, INC.**
**3055 Old Highway Eight P.O. Box 1453**
**Minneapolis Minnesota 55440 (US)**

(72) Inventor: **Thompson, David L.**
**1660 Onondaga Street**
**Fridley Minnesota 55432 (US)**
Inventor: **McDonald, Raymond S.**
**2241 Marion Street**
**St. Paul Minnesota 55113 (US)**
Inventor: **Lee, Yan Sang**
**14930 31st Avenue**
**Plymouth Minnesota 55441 (US)**
Inventor: **Stein, Marc T.**
**1865 East Broadway Road Apt. 153**
**Tempe Arizona 85282 (US)**

(74) Representative: **Tomlinson, Kerry John et al**
**Frank B. Dehn & Co. European Patent Attorneys**
**Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

Body implantable cardiac stimulators or pacemakers are known to the prior art. An early pacemaker is disclosed by Greatbatch in U.S. Patent No. 3,057,356, entitled "Medical Cardiac Pacemaker", which issued in 1962. This device included a relaxation oscillator that generated electrical pulses at a fixed rate. The pulses were applied to the heart to cause the heat to contract each time a pulse occurred.

Pacing technology has lagged behind conventional state of the art electronic technology in its utilization of digital electronic circuits. One reason for this has been the high energy required to operate digital electronic circuits. Energy requirements are a major concern in pacemaker design. However, with the continuing advances of electronic technology, digital electronic circuits are increasingly feasible within the context of commercial pacemaker units.

The accuracy and reliability of digital electronic circuits are factors that encourage their use within the pacemaker context. The facility with which they can be programmed and reprogrammed to alter one or more operating parameters further enhances their utility. For example, pacemakers have been disclosed which respond to magnetic and/or radio frequency signals to alter an operating parameter. Pulse rate and pulse width may be programmed in this manner. In addition, pacemakers have been constructed which are inhibited in the presence of certain signals, although no known prior art pacemaker is capable of having more than two parameters, features or tests programmed on command.

The implementation of digital electronic circuitry within the pacemaker context provides the opportunity to program or reprogram one or several operating parameters, on command, via externally generated signals. For example, pulse rate, pulse width and pulse amplitude can be externally established at one of any number of combinations. In addition, the refractory period may be established and altered. Further, digital circuitry can be programmed on a temporary or permanent basis, as desired. Of course, other operating parameters or characteristics can also be externally programmable.

Clearly then, a pacemaker utilizing digital electronic circuitry would have a more universal application by allowing the pacemaker to be programmed to fit the needs of a particular application as opposed to being manufactured for limited applications. In addition, such a unit can be instructed to give an external indication of its program status, particularly in instances where that status is not directly observable. However, even with the implementation of digital circuitry, certain analog circuitry is necessary to generate and/or transmit various control signals and to respond to the digital circuitry to effect its programming.

The present invention relates to analog circuitry intended for cooperation with digital circuitry to assist in the performance of the pacemaking function. Among the analog circuit functions necessary within the context of a digital pacemaker are the demodulation of a programming signal, the detection of heart activity during operation in a demand mode and provision of clock pulses. Additionally, analog circuitry may be employed to give an indication of battery status and to impose an upper rate limit on the simulation initiating signals generated by the digital circuit. The digital circuitry may provide a signal to control the sensitivity of the sense amplifier and a signal to establish a refractory period within the sense amplifier. The output analog circuit may be controlled by the digitial circuit to speed up the recharging of a capacitor in the output circuit, to establish the magnitude of the output pulses and to impose an upper rate limit on the output stimulation pulses.

During the delivery of a stimulation signal, the output capacitor is partially discharged and recharges during the interval between stimulation signals, the capacitor may not fully charge during that interval. This potential problem could be accommodated by reducing the time constant of the charge path of the output capacitor. However, in the context of a demand cardiac pacemaker it is desirable to have a high impedance in that path in that a high input impedance, as viewed from the heart, aids in sensing R waves. However, this higher impedance increases the time constant and, thus, the time of recharge. The sense amplifier of a demand cardiac pacemaker senses this activity and, thus, it would be desirable to speed up the recharge of the output capacitor to speed up the recovery of the sense amplifier. U.S. Patent 3,835,865 describes pacemaker output circuits in which the output capacitor is automatically recharged through a low impedance circuit including a diode or transistor which switches on at the end of the simulating pulse. However the recharge time is uncontrolled and depends among other things on the impedance of the body tissue so there is still a risk of recharge currents interfering with the sensing operation.

According to the invention, there is provided an implantable body stimulator comprising an output terminal adapted for connection to the body for the delivery of stimulation signals thereto and means for storing stimulation energy connected to said output terminal, a circuit for controlling discharge of the energy storing means into the body, and means forming a first charge path for said energy storing means, characterised by means forming a second, selectively conductive, charge path for said energy storing means, said second charge path having a lower impedance than said first charge path, and a charge control circuit for selectively rendering said second charge path conductive independently of said discharge controlling circuit and of the charge of the energy storing means.

The invention also provides an implantable body stimulator comprising a pair of output terminals adapted for connection to the body for the delivery of stimulation signals thereto; a capacitor one electrode of which is connected to one of said output terminals and the other electrode of which is connected via a first resistor to one pole of the stimulator power source, the other output terminal being connected to the other pole of the power source, whereby the capacitor charges from the power source; and a circuit controlling discharge of the capacitor into the body in response to stimulation pulse initiate signals; characterised by said other output terminal being connected to the other pole of the power source via a second resistor; a first transistor connected in parallel with said first resistor; a second transistor connected in parallel with said second resistor; and circuit means for causing said first and second transistors to conduct simultaneously to speed the charging of the capacitor, said circuit means being operable in response to recharge signals which are independent of said stimulation pulse initiate signals.

The invention provides an output circuit which provides a high input impedance, as viewed from the body tissue being stimulated, while increasing the charge rate of the output capacitor for a predetermined period following each stimulation pulse under the control of a RECHARGE signal generated by the digital circuitry.

Within the context of cooperating analog and digital circuitry for the generation and application of stimulating pulses, the preferred embodiment is directed to an output circuit for the provision of stimulation signals, the circuit being responsive to output initiate signals from the digital circuitry to establish the amplitude, duration and repetition rate of the simulation signals while providing a fast recharge of the output capacitor following a stimulation signal and establishing an upper rate limit for the generation of output initiate signals by the digital circuitry and stimulation signals by the output circuit. In addition, the output circuit is responsive to a signal from the digital circuitry to establish an independent upper rate limit on the generation of stimulation signals.

In accordance with its programming, the digital circuitry generates an output initiate signal in the form of a SINGLE or DOUBLE signal. Each output initiate signal results in the provision of a stimulation signal by the output circuit, the repetition rate and duration of the stimulation signal being established by the repetition rate and duration of the output initiate signal, respectively. The amplitude of the stimulation signal is dependent on the output initiate signal generated by the digital circuitry. For example, the generation of a DOUBLE signal results in a stimulation signal having an amplitude approximately twice the supply voltage. A SINGLE initiate signal results in a stimulation signal having an amplitude approximately equal to the supply voltage. Thus, the output circuit of the present invention will provide stimulation signals having an amplitude established by the output initiate signals generated by the digital circuitry. The ability to alter the stimulation signal amplitude, duration and repetition rate provides great flexibility in establishing the operating parameters during normal operation as well as during testing of the stimulator and its interaction with the body.

As indicated above, the output circuit of the present invention provides a stimulation signal in accordance with an output initiate signal generated by the digital circuitry. The repetition rate of the simulation signal is dependent on the repetition rate of the output initiate signal. Therefore, in the event that the digital circuitry should fail such that output initiate signals are generated at a rate higher than desirable the stimulation signal providing portion of the output circuit of the present invention would respond to those initiate signals and provide stimulation signals at a repetition rate higher than desirable. The output circuit of the present invention provides a rate limit circuit which prevents the response of the output circuit to the output initiate signals for a predetermined time after each output initiate signal thereby providing an upper rate limit to the stimulation signals. In addition, a RATE LIMIT signal is applied to the digital circuitry during that same period to prevent the generation of output initiate signals. The digital circuitry may be programmed to provide a rate limit disable signal to the output circuit, the output circuit responding to the rate limit disable signal from the digital circuitry to prevent the provision of the RATE LIMIT signal and to allow the output circuit to respond to all output initiate signals.

An exemplary embodiment of the invention will now be described with reference to the accompanying drawings, in which:

Figure 1 shows the interconnection and cooperation between the digital circuit of a cardiac pacemaker and a cooperating analog circuit of which the present invention relates to a part.

Figure 2 illustrates the operation of an output circuit forming a part of the analog circuit of Figure 1.

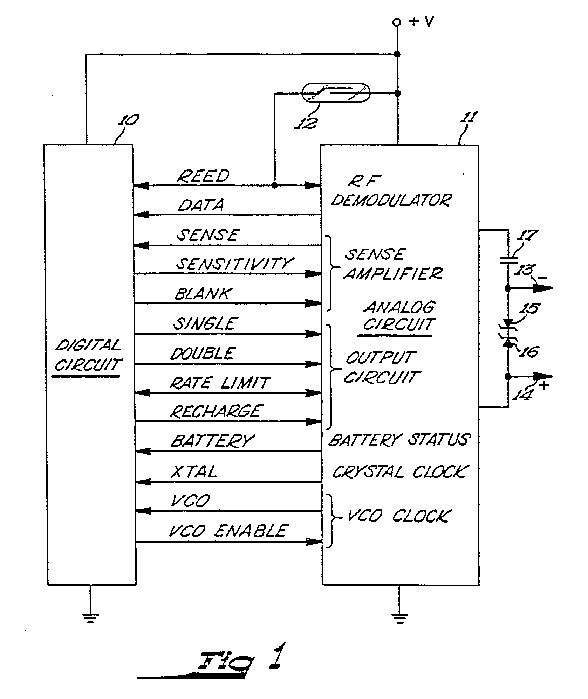

Referring now to Figure 1 there is shown a block diagram illustrating the interconnections between Digital Circuitry 10 (as more fully disclosed in our European Patent Specification 0011939 and Analog Circuitry 11 (of which the present invention reates to a part). Both the Digital Circuit 10 and Analog Circuit 11 are connected between a source of positive potential +V and a reference potential, such as ground. The source of positive potential may be a battery such as the conventional lithium iodide battery which generates approximately 2.8 volts.

The Analog Circuit 11 consists of various distinct electrical systems which may be referred to functionally as an RF Demodulator, a Sense Amplifier, an Output Circuit, a Battery Monitor and Status Indicator, a Crystal Clock and a Voltage Controlled Oscillator Clock. The Digital Circuit 10 includes all of the digital logic necessary to cause a programming change, memory to store the digital code manifesting the desired values for the program parameters and digital timing means for causing a stimulation pulse to be generated in the programmed manner. The signals applied between the Digital Circuit 10 and Analog Circuit 11 are REED, DATA, SENSE, SENSITIVITY, BLANK, SINGLE, DOUBLE, RATE LIMIT, RECHARGE, BATTERY, XTAL, VCO and VCO ENABLE.

A magnetically actuated reed relay switch 12 is connected between the source of positive potential +V and both the Digital Circuit 10 and the RF Demodulator of Analog Circuit 11. Reed switch 12 is normally open and is closed as by placing a magnet in close proximity thereto. When closed, a +V, or logic "1", REED signal is applied to both the Digital Circuit 10 and Analog Circuit 11. On removal of the magnet, the reed switch 12 opens and a ground, or logic "0", signal is applied to the Digital Circuit 10 and Analog Circuit 11. The RF Demodulator is enabled by a +V REED signal produced by a closing of the reed switch 12 to provide a DATA signal to the digital circuit 10. The DATA signal (the Digital Circuit 10 programming signal) is a pulse signal going from logic "0" to logic "1", as described in the specification referred to, which is representative of pulse bursts generated externally.

The Sense Amplifier portion of the analog circuit 11 provides a SENSE signal each time natural heart activity is detected to restart the timing cycle of the Digital Circuit 10, when operating in a demand mode. A SENSITIVITY signal is provided by the Digital Circuit 10 in accordance with its programming to establish the detection level of the Sense Amplifier. A BLANK signal is generated by the Digital Circuit 10 and applied to the Sense Amplifier portion of the Analog Circuit 11 to establish the refractory period of the Sense Amplifier and to allow the components within the Sense Amplifier to reset themselves.

The Output Circuit of analog circuit 11 includes output terminals 13 and 14 which are adapted for connection to a conventional lead, in a known manner. The output terminal 14 may be connected to a metal casing housing the pacemaker unit or a plate forming a part of the casing in a unipolar lead system or it may be connected to a second lead in a bipolar lead system, depending on the type of lead system employed. Output terminal 13 is coupled through a capacitor 17 to the analog Output Circuit and to the heart (not shown). In addition, a pair of Zener diodes 15 and 16 have their anodes coupled together and their cathodes coupled to output terminals 13 and 14, respectively. Diodes 15 and 16 function in a conventional manner to prevent damage to the pacemaker circuitry in the presence of large extraneous signals such as are caused by electrocautery. The Output Circuit of Analog Circuit 11 includes elements responsive to a SINGLE or DOUBLE signal from Digital Circuit 10 to control the amplitude of output signals applied across output terminals 13 and 14. A RECHARGE signal from Digital Circuit 10 speeds up the recharging of output capacitor 17 while the Output Circuit of Analog Circuit 11 provides a RATE LIMIT signal to Digital Circuit 10 to provide an upper limit to the rate at which stimulation initiating signals are generated. Digital circuit 10 also provides a rate limit disable signal to the Output Circuit of Analog Circuit 11 to eliminate the upper limit to the rate at which stimulation pulses may be applied by the Output Circuit.

In addition to the above, Analog Circuit 11 includes circuitry which monitors the status of the battery to provide an indication of that status in the form of the signal BATTERY. Also, clock pulses are provided to the Digital Circuit 10 in the form of signals XTAL and VCO. Within the context of the Digital Circuit of the specification referred to, the XTAL signal is a generally square wave pulse signal occurring at a frequency of 32,768 Hz and the VCO signal is a square wave pulse signal having a preset frequency whenever +V is equal to 2.8 volts. As +V decreases with time, as the battery depletes, the frequency of the VCO signal will also decrease, in known manner. The VCO signal is used in the timing circuitry of Digital Circuit 10 to establish the exact width of stimulating pulse. In order to maintain a constant energy of this pulse, it is necessary that the pulse increase in width as +V decreases. The VCO clock pulse generator is enabled only during the time the stimulating pulse is to be provided and is enabled by the signal VCO ENABLE.

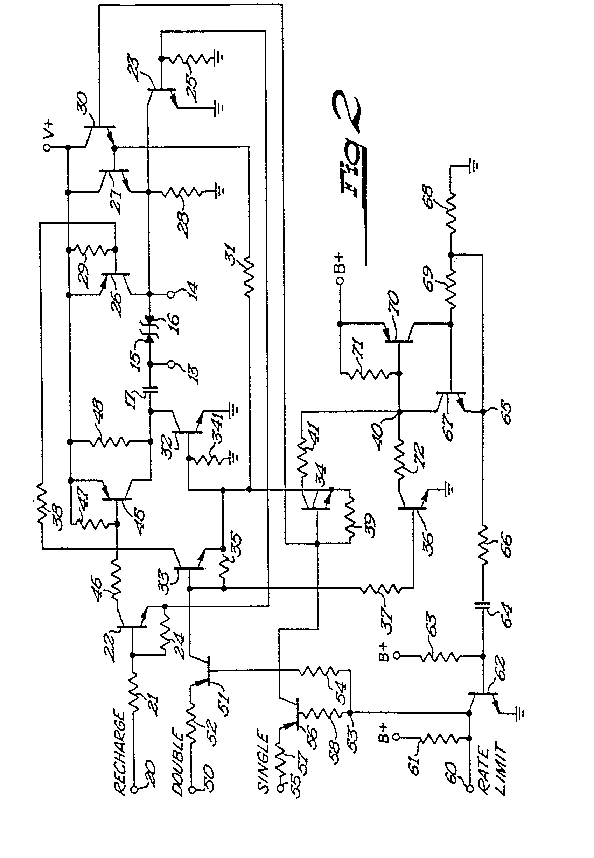

Referring now to Figure 2, there is shown a preferred embodiment of the output circuit of the present invention with elements 13—17 of Figure 2 being those of like reference numeral illustrated in Figure 1. A terminal 20 is adapted to receive the RECHARGE signal from digital circuit 10 of Figure 1 and is connected to a resistor 21. Resistor 21 is connected to the base of a transistor 22 and to the emitter of transistor 22 and the base of transistor 23 via resistor 24. The base of transistor 23 is connected to ground via resistor 25 while its emitter is connected to ground and its collector is connected to terminal 14 and to the collector of transistor 26 and the emitter of transistor 27. The emitter of transistor 27 is connected to ground via resistor 28 while its collector and the emitter of transistor 26 are connected to a positive potential V+. A resistor 29 connects the base of transistor 26 to V+. A transistor 30 has its collector connected to V+ while its emitter is connected to the base of transistor 27 and to a resistor 31. The resistor 31 is connected to the base of a transistor 32, the emitter of transistor 33 and the emitter of a transistor 34. The base of transistor 32 is connected to ground via resistor 341 while its emitter is connected to ground and its collector is connected to capacitor 17. The emitter of transistor 33 is connected to its base via resistor 35 and to the base of a transistor 36 via resistor 37. A resistor 38 connects the collector of transistor 33 to the base of transistor 26. The emitter of transistor 34 is connected via resistor 39 to its base and to the base of transistor 30 while its collector is connected to a junction 40 via resistor 41.

The collector of transistor 22 is connected to the base of a transistor 45 via a resistor 46, the base and emitter of transistor 45 being connected via resistor 47. A resistor 48 interconnects the emitter and collector of transistor 45, the collector of transistor 45 being connected to the capacitor 17 and the emitter to V+.

A terminal 50 is adapted for connection to receive a DOUBLE signal from digital circuit 10 and is connected to the emitter of a transistor 51 via a resistor 52. The collector of transistor 51 is connected to the base of transistor 33 while its base is connected to a junction 53 via resistor 54. A terminal 55 is adapted for connection to receive the SINGLE signal from the digital circuit 10 and is connected to the emitter of a transistor 56 via resistor 57. The collector of transistor 56 is connected to the base of transistor 34 while its base its connected to junction 53 via resistor 58. A terminal 60 is adapted for connection to receive the RATE LIMIT signal from digital circuit 10 and is connected to a positive potential B+ via resistor 61 and to the collector of a transistor 62 and the junction 53. The emitter of transistor 62 is connected to ground and its base is connected to B+ via a resistor 63 and to a capacitor 64. The capacitor 64 is connected to a junction 65 via a resistor 66, the junction 65 being connected to the emitter of a transistor 67, to ground via a resistor 68 and to the base of transistor 67 via a resistor 69. The base of transistor 67 is connected to the collector of a transistor 70 while its collector is connected to the junction 40. The emitter of transistor 70 is connected to B+ and to its base and junction 40 via a resistor 71. The collector of transistor 36 is connected to junction 40 via resistor 72. As described above, V+ is a source of positive potential. B+ indicates a positive potential source that is filtered to prevent the ripple caused by a stimulation pulse from turning on the rate limit circuitry in the middle of a stimulation pulse and thus cause a loss of a part of the stimulation pulse.

As outlined above, a DOUBLE signal appearing at terminal 50 will result in a stimulation signal at terminals 13 and 14 approximately twice the potential of V+. The DOUBLE signal is a positive pulse having a duration that is essentially the duration of the desired stimulation pulse. This pulse turns on transistor 51 resulting in the turn on of transistor 33. The turn on of transistor 33 causes transistors 26 and 32 to saturate. Assuming that capacitor 17 had charged to V+, the turn on of transistor 32 connects the left or positive side of capacitor 17 to ground driving its right side and terminal 13 negative. The turn on of transistor 26 connects terminal 14 to V+ and, accordingly, the voltage across terminal 13 and 14 is V+ —(—V+) = 2V+. In practice, of course, saturation losses will result in a slightly lower voltage applied across the terminals 13 and 14.

The SINGLE signal applied to terminal 55 is a positive pulse having a duration essentially that of the desired stimulation pulse. This signal turns on transistor 56 which turns on transistors 34 and 30. The turn on of transistor 34 again causes transistor 32 to saturate again forcing the terminal 13 negative. Transistor 30 turns on transistor 27. However, the emitter of transistor 27 is clamped at ground potential due to the fact that the base emitter voltage of transistor 34 plus that of transistor 32 must equal the base emitter voltage of transistor 30 plus that of transistor 27. Thus, terminal 14 is maintained at ground potential and approximately the negative of V+ is applied across the terminals 13 and 14. Again, of course, the saturation losses in transistor 32 reduce the potential across the terminals 13 and 14 by a small amount.

During the stimulation pulse resulting from the SINGLE output initiate signal, transistor 32 is saturated while transistor 27 is in its linear range. Thus, at the end of the SINGLE signal, transistor 27 would turn off faster than transistor 32. However, terminal 13 would still be negative when transistor 27 turns off resulting in a path for current flow through the body tissue connected between terminals 13 and 14 and resistor 28 producing a negative spike on the collector of transistor 23. Resistor 31 eliminates this spike by providing a low impedance path for the base of transistor 32 which allows transistor 32 and 27 to turn off simultaneously.

During the delivery of a stimulation signal, capacitor 17 is partially discharged — typically on the order of 0.5 volt. A short time after the stimulation signal has ended the digital circuitry 10 provides a RECHARGE signal in the form of a positive pulse applied to terminal 20. The RECHARGE signal has a pulse width of approximately 10 milliseconds and turns on transistor 22. The turn on of transistor 22 causes transistors 45 and 23 to saturate allowing capacitor 17 to charge quickly through transistor 23, the body tissue connected between terminals 13 and 14 and transistor 45. During this fast recharge interval (the duration of the RECHARGE Signal) capacitor 17 charges to approximately V+ less saturation losses in transistors 23 and 45. Those transistors may be selected to minimize the saturation losses and thus maximize the charge on capacitor 17. Resistors 28 and 48 allow the continued charging of capacitor 17 after the fast recharge interval until the next output pulse is initiated. Thus, the output circuit provides means for increasing the charge rate of the output capacitor by providing first and second charge paths for the capacitor, one charge path being selectively conductive and of a lower impedance than the other path. In the illustrated embodiment, transistors 23 and 45 provide shunts across resistances within a normal capacitor charge path thereby reducing the impedance and time constant of the charge path during the time that the RECHARGE signal is applied to the terminal 20. A high impedance is maintained at all other times as an aid in sensing R waves.

Transistor 62 is normally saturated resulting in a "zero" or ground condition at terminal 60. A positive signal applied to the terminal 60 disables the transistors 51 and 56 and prevents them from turning on in response to an output initiate signal. In essence, such a condition blocks the output initiate signals. Within the Output Circuit a positive signal on terminal 60 is obtained, via resistor 61, when transistor 62 is 'off'. Turn on of transistor 62 causes its collector to go to ground potential thus enabling transistors 51 and 56 and, accordingly, the rest of the output circuit of Figure 2. The signal at terminal 60 resulting from the turn on and turn off of transistor 62 may also be applied as the RATE LIMIT signal to the digital circuit 10 to enable and disable the generation of output initiate signals. Accordingly, the turn off of transistor 62 may be employed as a disable signal in the digital circuit 10 to prevent the generation of output initiate signals thereby providing an additional upper rate limit.

The appearance of a SINGLE or DOUBLE output initiate signal results in a signal at junction 40 and a turn on of transistors 67 and 70. For example, a DOUBLE signal at terminal 50 will turn on transistor 51 resulting in a turn on of transistor 36 and a signal at junction 40. A SINGLE signal at terminal 55 will turn on transistor 56 resulting in a turn on of transistor 34 and a signal at junction 40. In either event, a signal at junction 40 results in the turn on of transistors 67 and 70 which are connected in an SCR arrangement. Once the circuit of transistors 67 and 70 is triggered, capacitor 64 will charge and transistors 67 and 70 will remain on until capacitor 64 is charged and the SINGLE or DOUBLE output initiate signal terminates. Thus, the on time of transistors 67 and 70 is established by the duration of the output initiate signal with the charge time of capacitor 64 setting a minimum on time. On termination of the SINGLE or DOUBLE output initiate signal, with capacitor 64 charged, the transistors 67 and 70 will turn off returning the right side of capacitor 64 to ground via resistors 66 and 68. Since the voltage across capacitor 64 cannot change instantaneously, the base of transistor 62 is driven negative cutting it off and causing its collector to go positive. This positive signal at the collector of transistor 62 disables the transistors 51 and 56 and may be employed as a RATE LIMIT signal within the analog circuit 10. With transistors 67 and 70 off, capacitor 64 charges toward B+ through resistors 63, 66 and 68 until the base of transistor 62 is forward biased. At that time, transistor 62 turns on putting a zero condition at terminal 60, again enabling transistors 51 and 56. The maximum output stimulation rate is thus limited by the time that transistor 62 is off, that time being established by the time constant of the circuitry including capacitor 64 and resistors 63, 66 and 68 and being selectable at any desired rate, in known manner. It should be noted that the transistors 67 and 70 stay on for at least the duration of a SINGLE or DOUBLE output initiate signal which prevents the turn off of transistor 62 during those signals and, thus, prevents the disruption of a stimulation signal.

When the transistor 62 is non-conductive, thus blocking the output initiate signals with a positive voltage at junction 53, a low signal supplied to terminal 60 from the digital circuitry 10 will override the blocking signal thus allowing a higher rate of stimulation than that established by the time constant referred to.

As described above, the present invention provides an output circuit within the context of cooperating analog and digital circuitry for the generation and application of stimulating signals. The output circuit allows a selection of stimulation signal amplitudes, provides a speed up in the charge rate of the output capacitor and imposes a rate limit on both itself and the elements generating the output initiate signals. However, it is to be understood that the concepts underlying the several aspects of the present invention need not be confined to a digital/analog combination or to the specific embodiment illustrated. In particular the output circuit may be employed in apparatus for stimulating parts of the body other than the heart. It is therefore to be understood that, within the scope of the appended claims, the invention may be practised otherwise than as specifically described.

**Claims**

1. An implantable body stimulator comprising an output terminal (13) adapted for connection to the body for the delivery of stimulation signals thereto and means (17) for storing stimulation energy connected to said output terminal, a circuit (56, 34, 32, 30, 27, 51, 36, 26) for controlling discharge of the energy storing means into the body, and means (48, 28) forming a first charge path for said energy storing means, characterised by means (45, 23) forming a second, selectively conductive, charge path for said energy storing means, said second charge path having a lower impedance than said first charge path, and a charge control circuit (22) for selectively rendering said second charge path conductive independently of said discharge controlling circuit and of the charge of the energy storing means.

2. A stimulator as claimed in claim 1 characterised in that said first charge path comprises resistance means (48, 28) and said second charge path comprises electronic switch means (45, 23) shunted across said resistance means and controllable so as to be selectively rendered conductive.

3. A stimulator as claimed in claim 1 or 2 in the form of a programmable pacer including a source of supply voltage; a pair of output terminals (13, 14) adapted to be connected to body tissue; a digital circuit (10) adapted to be remotely programmed to periodically provide output initiate signals and recharge signals recurring in sequence at times determined by the stored program values; said discharge controlling circuit being responsive to said output initiate signals for providing corresponding stimulating pulse signals at said output terminals; characterised by said first charge path forming means (48, 28) being coupled to said voltage source for recharging said energy storing means (17) over the time interval between output initiate signals; and said second charge path forming means (45, 23) being coupled to said voltage source and responsive to said recharge signals for rapidly recharging said energy storing means (17) while said recharge signal persists.

4. A stimulator as claimed in any preceding claim characterised in that the stimulation energy storing means is a capacitor (17) one electrode of which is connected to an output terminal (13) of the stimulator and the other electrode of which is connected to one pole of the stimulator power source via a first resistance (48), another output terminal (14) being connected via a second resistance (28) to the other pole of the power source, and including respective transistors (45, 23) connected in parallel with said first and second resistors (48, 28) and arranged to be operated simultaneously to render said second charge path conductive.

5. An implantable body stimulator comprising a pair of output terminals (13, 14) adapted for connection to the body for the delivery of stimulation signals thereto; a capacitor (17) one electrode of which is connected to one (13) of said output terminals and the other electrode of which is connected via a first resistor (48) to one pole of the stimulator power source, the other output terminal (14) being connected to the other pole of the power source, whereby the capacitor (17) charges from the power source; and a circuit (56, 34, 32, 30, 27, 51, 33, 26) controlling discharge of the capacitor (17) into the body in response to stimulation pulse initiate signals; characterised by said other output terminal (14) being connected to the other pole of the power source via a second resistor (28); a first transistor (45) connected in parallel with said first resistor (48); a second transistor (23) connected in parallel with said second resistor (28); and circuit means (22) for causing said first and second transistors (48, 23) to conduct simultaneously to speed the charging of the capacitor (17), said circuit means (22) being operable in response to recharge signals which are independent of said stimulation pulse initiate signals.

6. A stimulator as claimed in claim 4 or 5 characterised by means (10) for selectively providing one of first and second output initiate signals, first means (56, 34, 32, 30, 27) arranged in response to the first initiate signals to couple said other electrode and the second output terminal (14) to the potential of the other pole of the power supply and second means (51, 33, 32, 26) arranged in response to the second initiate signals to couple said other electrode to said other pole potential and the second output terminal (14) to the potential of said one pole of the power supply, whereby output signals are provided to the output terminals (13, 14) of substantially the voltage of the power supply or substantially twice the voltage of the power supply in response to said first or second initiate signals respectively.

7. A stimulator as claimed in any preceding claim characterised by an output circuit for providing body-stimulating output signals, a circuit (10) for providing to the output circuit output initiate signals when a stimulating output signal is required, and means (67, 70, 62) for disabling the output circuit for a predetermined period after an output initiate signal.

8. A stimulator as claimed in claim 7 characterised in that said disabling means comprises means (62) for blocking said output initiate signals from the output circuit.

9. A stimulator as claimed in claim 7 or 8 characterised in that said disabling means (67, 70, 62) is also arranged to disable the initiate signals providing circuit.

10. A stimulator as claimed in claim 7, 8 or 9 characterised in that said disabling means includes an electronic switch (67, 70) controllable by a disable signal to block the provision of the initiate signals to the output circuit, said disable signal being provided in response to, but after, an initiate signal.

11. A stimulator as claimed in claim 10 characterised in that said electronic switch (67, 70) is also controllable into its non-blocking condition by a signal from the initiate signal providing circuit whereby the blocking effect of the disabling means may be overridden.

**Revendications**

1. Stimulateur implantable dans le corps, comprenant une borne de sortie (13) destinée à être connectée au corps pour lui délivrer des signaux de stimulation et un dispositif (17) pour emmagasiner de l'énergie de stimulation, connecté à ladite borne de sortie, un circuit (56, 34, 32, 30, 27, 51, 36, 26) pour contrôler la décharge du dispositif d'emmagasinage d'énergie dans le corps et un dispositif (48, 28) formant un premier circuit de charge pour ledit dispositif d'emmagasinage d'énergie, caractérisé par un dispositif (45, 23) formant un second circuit de charge sélectivement conductif pour ledit dispositif d'emmagasinage d'énergie, ledit second circuit de charge ayant une impédance inférieure à celle dudit premier circuit de charge et un circuit de commande de charge (22) pour rendre sélectivement conducteur ledit second circuit de charge indépendamment dudit circuit de commande de décharge et de la charge du dispositif d'emmagasinage d'énergie.

2. Stimulateur selon la revendication 1, caractérisé en ce que ledit premier circuit de charge consiste en une résistance (48, 28) et ledit second circuit de charge consiste en un commutateur électronique (45, 23) connecté en dérivation sur ladite résistance et pouvant être commandé de manière à être rendu conducteur sélectivement.

3. Stimulateur selon la revendication 1 ou 2, sous la forme d'un stimulateur programmable comprenant une source de tension d'alimentation; une paire de bornes de sortie (13, 14) adaptées pour être connectées à des tissus du corps, un circuit numérique (10) adapté pour être programmé à distance pour produire périodiquement des signaux de déclenchement de sortie et des signaux de recharge apparaissant répétitivement dans une séquence à des instants déterminés par les valeurs de programme mémorisées; ledit circuit de commande de décharge réagissant auxdits signaux de déclenchement de sortie en produisant des signaux d'impulsions de stimulation correspondants auxdites bornes de sortie; caractérisé en ce que ledit premier dispositif formant un circuit de charge (48, 28) est couplé avec ladite source de tension pour recharger ledit dispositif d'emmagasinage d'énergie (17) dans l'intervalle de temps entre des signaux de déclenchement de sortie; et ledit second dispositif formant un circuit de charge (45, 23) étant couplé avec ladite source de tension et réagissant auxdits signaux de recharge pour recharger rapidement ledit dispositif d'emmagasinage d'énergie (17) pendant que ledit signal de recharge persiste.

4. Stimulateur selon l'une quelconque des revendications précédentes, caractérisé en ce que le dispositif d'emmagasinage d'énergie de stimulation est un condensateur (17) dont une électrode est connectée à une borne de sortie (13) du stimulateur et dont l'autre électrode est connectée à un pôle de la source d'alimentation du stimulateur par une première résistance (48), une autre borne de sortie (14) est connectée par une seconde résistance (28) à l'autre pôle de la source d'alimentation et comprenant des transistors respectifs (45, 23) connectés en parallèle avec ladite première et ladite seconde résistances (48, 28) et étant agencés pour être commandés simultanément pour rendre conducteur ledit second circuit de charge.

5. Stimulateur implantable dans le corps, comprenant une paire de bornes de sortie (13, 14) adaptées pour être connectées au corps pour lui délivrer des signaux de stimulation; un condensateur (17) dont une électrode est connectée à l'une (13) desdites bornes de sortie et dont l'autre électrode est connectée par une première résistance (48) à un pôle de la source d'alimentation du stimulateur, l'autre borne de sortie (14) étant connectée à l'autre pôle de la source d'alimentation, de manière que le condensateur (17) soit chargé par la source d'alimentation, et un circuit (56, 34, 32, 30, 27, 51, 33, 26) commandant la décharge du condensateur (17) dans le corps en réponse à des signaux de déclenchement d'impulsions de stimulation; caractérisé en ce que ladite autre borne de sortie (14) est connectée à l'autre pôle de la source d'alimentation pour une seconde résistance (28); un premier transistor (45) connecté en parallèle avec ladite première résistance (48); un second transistor (23) connecté en parallèle avec ladite seconde résistance (28) et un circuit (22) pour que ledit premier et ledit second transistors (48, 23) conduisent simultanément pour accélérer la charge du condensateur (17), ledit circuit fonctionnant en réponse à des signaux de recharge qui sont indépendants desdits signaux de déclenchement d'impulsions de stimulation.

6. Stimulateur selon la revendication 4 ou 5, caractérisé en ce qu'il comporte un dispositif (10) pour produire sélectivement l'un du premier et du second signaux de déclenchement de sortie, un premier dispositif (56, 34, 32, 30, 27) agencé pour en réponse aux premiers signaux de déclenchement, coupler ladite autre électrode et ladite seconde borne de sortie (14) au potentiel de l'autre pôle de la source d'alimentation et un second dispositif (51, 33, 32, 26) agencé pour en réponse aux seconds signaux de déclenchement, coupler ladite autre électrode audit autre potentiel de pôle et la seconde borne de sortie (14) au potentiel dudit pôle de la source d'alimentation de manière que des signaux de sortie soient produits aux bornes de sortie (13, 14), pratiquement à la tension de la source d'alimentation ou pratiquement au double de la tension de la source d'alimentation en réponse audit premier ou audit second signaux de déclenchement respectivement.

7. Stimulateur selon l'une quelconque des revendications précédentes, caractérisé par un circuit de sortie qui produit des signaux de sortie de stimulation du corps, un circuit (10) pour fournir au circuit de sortie des signaux de déclenchement de sortie lorsqu'un signal de sortie de stimulation est nécessaire et un dispositif (67, 70, 62) pour inhiber le circuit de sortie pendant une période prédéterminée après un signal de déclenchement de sortie.

8. Stimulateur selon la revendication 7, caractérisé en ce que ledit dispositif d'inhibition consiste en un dispositif (62) qui bloque lesdits signaux de déclenchement de sortie pour ledit circuit de sortie.

9. Stimulateur selon la revendication 7 ou 8, caractérisé en ce que ledit dispositif d'inhibition (67, 70, 62) est également agencé pour inhiber le circuit produisant des signaux de déclenchement.

10. Stimulateur selon la revendication 7, 8 ou 9, caractérisé en ce que ledit circuit d'inhibition comporte un commutateur électronique (67, 70) pouvant être commandé par un signal d'inhibition pour bloquer la production des signaux de déclenchement pour le circuit de sortie, ledit signal d'inhibition étant produit en réponse au signal de déclenchement, mais après lui.

11. Stimulateur selon la revendication 10, caractérisé en ce que ledit commutateur électronique (67, 70) peut également être commandé dans sa condition de non blocage par un signal provenant du circuit produisant un signal de déclenchement de manière que l'effet de blocage du dispositif d'inhibition puisse être dépassé.

## Patentansprüche

1. Implantierbarer Körperstimulator mit einem an den Körper anschließbaren Ausgangsanschluß (13) für die Abgabe von Reizsignalen an den Körper, einer mit dem Ausgangsanschluß verbundenen Anordnung (17) zum Speichern von Reizenergie, einer Schaltungsanordnung (56, 34, 32, 30, 27, 51, 36, 26) zum Steuern der Entladung der Energiespeicheranordnung in den Körper, und einer einen ersten Ladeweg für die Energiespeicheranordnung bildenden Anordnung (48, 28), gekennzeichnet durch eine einen zweiten selektiv leitfähigen Ladeweg für die Energiespeicheranordnung bildende Anordnung (45, 23), wobei der zweite Ladeweg eine niedrigere Impedanz als der erste Ladeweg hat, sowie eine Ladesteuerschaltung (22), die den zweiten Ladeweg unabhängig von der Entladesteuerschaltungsanordnung und von der Ladung der Energiespeicheranordnung selektiv leitfähig macht.

2. Stimulator nach Anspruch 1, dadurch gekennzeichnet, daß der erste Ladeweg eine Widerstandsanordnung (48, 28) aufweist und der zweite Ladeweg mit einer elektronischen Schaltanordnung (45, 23) versehen ist, die parallel zu der Widerstandsanordnung liegt und derart steuerbar ist, daß sie selektiv leitfähig gemacht wird.

3. Stimulator nach Anspruch 1 oder 2 in Form eines programmierbaren Schrittmachers mit einer Versorgungsspannungsquelle; zwei an Körpergewebe anschließbaren Ausgangsanschlüssen (13, 14); einer digitalen Schaltungsanordnung (10), die fernprogrammierbar ist, um periodisch Ausgangsauslösesignale und Wiederladesignale anzuliefern, die der Reihe nach zu Zeitpunkten wiederkehren, die von den gespeicherten Programmwerten bestimmt sind; wobei die Entladesteuerschaltungsanordnung auf die Ausgangsauslösesignale anspricht, um entsprechende Reizimpulssignale an den Ausgangsanschlüssen bereitzustellen, dadurch gekennzeichnet, daß die den ersten Ladeweg bildende Anordnung (48, 28) mit der Spannungsquelle zum Wiederaufladen der Energiespeicheranordnung (17) während des Zeitintervalls zwischen Ausgangsauslösesignalen gekoppelt ist; und daß die den zweiten Ladeweg bildende Anordnung (45, 23) an die Spannungsquelle angekoppelt ist und auf die Wiederladesignale anspricht, um die Energiespeicheranordnung (17) rasch wiederaufzuladen, während das Wiederladesignal vorliegt.

4. Stimulator nach einem der vorhergehenden Ansprüche, dadurch gekennzeichnet, daß die Reizenergiespeicheranordnung ein Kondensator (17) ist, dessen eine Elektrode mit einem Ausgangsanschluß (13) des Stimulators verbunden ist und dessen andere Elektrode mit dem einen Pol der Stromquelle des Stimulators über einen ersten Widerstand (48) verbunden ist, daß ein weiterer Ausgangsanschluß (14) über einen zweiten Widerstand (28) mit dem anderen Pol der stromquelle verbunden ist, und daß Transistoren (45, 23) vorgesehen sind, die mit dem ersten bzw. dem zweiten Widerstand (48, 28) parallelgeschaltet und derart angeordnet sind, daß sie gleichzeitig betätigt werden, um den zweiten Ladeweg leitfähig zu machen.

5. Implantierbarer Körperstimulator mit zwei an den Körper anschließbaren Ausgangsanschlüssen (13, 14) für die Abgabe von Reizsignalen an den Körper; einem Kondensator (17), dessen eine Elektrode mit einem (13) der Ausgangsanschlüsse verbunden ist und dessen andere Elektrode über einen ersten Widerstand (48) mit einem Pol der Stimulatorstromquelle verbunden ist, während der andere Ausgangsanschluß (14) mit dem anderen Pol der Stromquelle verbunden ist, wodurch sich der Kondensator (17) aus der Stromquelle auflädt; und einer Schaltrungsanordnung (56, 34, 32, 30, 27, 51, 33, 26), welche das Entladen des Kondensators (17) in den Körper aufgrund von Reizimpulsauslösesignalen steuert; dadurch gekennzeichnet, daß der andere Ausgangsanschluß (14) mit dem anderen Pol der Stromquelle über einen zweiten Widerstand (28) verbunden ist; ein erster Transistor (45) parallel zu dem ersten Widerstand (48) geschaltet ist; ein zweiter Transistor (23) parallel zu dem zweiten Widerstand (28) geschaltet ist; und eine Schaltungsanordnung (22) vorgesehen ist, welche den ersten und den zweiten Transistor (48, 23) veranlaßt, gleichzeitig zu leiten, um das Aufladen des Kondensators (17) zu beschleunigen, wobei die Schaltungsanordnung (22) aufgrund von Wiederladesignalen betätigbar ist, die von den Reizimpulsauslösesignalen unabhängig sind.

6. Stimulator nach Anspruch 4 oder 5, gekennzeichnet durch eine Anordnung (10) zum selektiven Anliefern eines von ersten und zweiten Ausgangsauslösesignalen, eine erste Anordnung (56, 34, 32, 30, 27), die so ausgelegt ist, daß sie aufgrund der ersten Auslösesignale die andere Elektrode und den zweiten Ausgangsanschluß (14) mit dem Potential des anderen Pols der Stromquelle koppelt, und eine zweite Anordnung (51, 33, 32, 26), die so ausgelegt ist, daß sie aufgrund der zweiten Auslösesignale die andere Elektrode mit dem Potential des anderen Pols und den zweiten Ausgangsanschluß (14) mit dem Potential des einen Pols der Stromquelle koppelt, wodurch an die Ausgangsanschlüsse (13, 14) Ausgangssignale mit im wesentlichen der Spannung der Stromquelle oder im wesentlichen dem Zweifachen der Spannung der Stromquelle aufgrund der ersten bzw. der zweiten Auslösesignale angeliefert werden.

7. Stimulator nach einem der vorhergehenden Ansprüche, gekennzeichnet durch eine Ausgangsschaltung zum Bereitstellen von Körperreiz-Ausgangssignalen, eine Schaltungsanordnung (10), die der Ausgangsschaltung Ausgangsauslösesignale zuführt, wenn ein Reizausgangssignal benötigt wird, und eine Anordnung (67, 70, 62) zum Sperren der Ausgangsschaltung für eine vorbestimmte Zeitspanne nach einem Ausgangsauslösesignal.

8. Stimulator nach Anspruch 7, dadurch gekennzeichnet, daß die Sperranordnung Mittel (62) zum Absperren der Ausgangsauslösesignale von der Ausgangsschaltung aufweist.

9. Stimulator nach Anspruch 7 oder 8, dadurch gekennzeichnet, daß die Sperranordnung (67, 70, 62) ferner so ausgelegt ist, daß sie die die Auslösesignale bereitstellende Schaltungsanordnung sperrt.

10. Stimulator nach Anspruch 7, 8 oder 9, dadurch gekennzeichnet, daß die Sperranordnung einen elektronischen Schalter (67, 70) aufweist, der mittels eines Sperrsignals steuerbar ist, um die Anlieferung der Auslösesignale an die Ausgangsschaltung zu sperren, wobei das Sperrsignal im Ansprechen auf, aber nach einem Auslösesignal angeliefert wird.

11. Stimulator nach Anspruch 10, dadurch gekennzeichnet, daß der elektronische Schalter (67, 70) ferner in seinen nichtsperrenden Zustand mittels eines Signals von der das Auslösesignal bereitstellenden Schaltungsanordnung überführbar ist, wodurch der Sperreffekt der Sperranordnung übersteuert werden kann.

Fig 1

Fig 2